# EUROPÄISCHE PATENTSCHRIFT

(11) **EP 2 275 019 B1**
(45) Veröffentlichungstag und Bekanntmachung des Hinweises auf die Patenterteilung: **11.09.2013**
(21) Anmeldenummer: 10007117.4
(22) Anmeldetag: 09.07.2010
(51) Int. Cl.: A61B 1/313

(54) **Ballon-Laparoskop, Trokarhülse und Verschlusseinsatz für eine Trokarhülse**
Balloon laparoscope, trocar sheath and fastening insert for a trocar sheath
Laparoscope à ballon, gaine de trocart et insert de fermeture pour une gaine de trocart

(30) Priorität: 15.07.2009 DE 102009033314
(43) Veröffentlichungstag der Anmeldung: 19.01.2011
(73) Patentinhaber: Pajunk Gmbh & Co. Kg, 78187 Geisingen (DE)
(72) Erfinder: Pajunk, Heinrich, 78187 Geisingen (DE); Pajunk, Horst, 78187 Geisingen (DE)
(74) Vertreter: Manitz, Finsterwald & Partner GbR

(56) Entgegenhaltungen:
- WO-A2-2008/028022
- US-A1- 2007 083 217
- US-A1- 2009 143 640

## Beschreibung

Die vorliegende Erfindung betrifft ein Ballon-Laparoskop für diagnostische und/oder minimal-invasive Eingriffe mit einem rohrförmigen Körper, an dessen distalen Endbereich ein Ballon befestigt ist, wobei das Innere des Ballons mit dem Lumen des rohrförmigen Körpers in Fluidverbindung steht, so dass der Ballon durch Zufuhr von Fluid über das Lumen des rohrförmigen Körpers in einen expandierten Zustand gebracht werden kann. Weiterhin ist die Erfindung auf eine Trokarhülse gerichtet, die zum Einführen eines Laparoskops oder eines Instruments für die minimal-invasive Chirurgie in den Abdomen ausgebildet ist, sowie auf einen Verschlusseinsatz für eine solche Trokarhülse.

Bei der heutzutage in der klinischen Praxis üblichen Laparoskopie wird über einen minimal-invasiven Schnitt in der Bauchdecke eine Trokarhülse eingeführt, durch die anschließend ein spezielles Endoskop (Laparoskop) in den Bauchraum eingeschoben wird. Das Laparoskop kann an eine Videokamera sowie an eine Lichtquelle angeschlossen werden, so dass der Bauchraum unterhalb der Bauchdecke über das Laparoskop eingesehen werden kann. Um ein für die Beobachtung ausreichendes, freies Sichtfeld zu erreichen, wird üblicherweise der Bauchraum mit unter Druck stehendem Kohlendioxidgas (CO₂) befüllt, bis ein Pneumoperitoneum geschaffen ist. Ohne Schaffung eines solchen Pneumoperitoneums würde die in den Bauchraum eingeführte Optik unmittelbar an den Organen zur Anlage kommen, so dass keine für die diagnostischen Zwecke erforderliche Bildwiedergabequalität erreichbar wäre.

Nachteilig an der Erzeugung eines Pneumoperitoneums ist es, dass das Aufpumpen des Baumraums mit unter Druck stehendem Gas für den Patienten belastend ist und in der Regel eine Vollnarkose erfordert. Auch der relativ große apparative Aufwand zur Durchführung dieser Operation ist nachteilig, da aufgrund des apparativen Aufwands eine solche Operation nur in einem voll ausgerüsteten Operationssaal durchgeführt werden kann.

Um eine Laparoskopie auch ohne Erzeugung eines Pneumoperitoneums durchführen zu können, wurden die eingangs genannten Ballon-Laparoskope entwickelt. Bei einem Ballon-Laparoskop ist das distal angeordnete optische Bildübertragungssystem von einem aufblasbaren Ballon aus klar durchsichtigem Material umgeben, der mit einem ebenfalls klar durchsichtigem Druckmedium (Gas oder Kochsalzlösung) aufpumpbar ist. Nach Aufpumpen des Ballons unterhalb der Bauchdecke im Bauchraum auf seinen vordefinierten expandierten Zustand wird entlang den Wänden des Ballons ein Panorama der unmittelbaren Umgebung des Ballons sichtbar. Alternativ kann ein zu visualisierender Bereich auch durch mechanische Gestänge aufgespannt werden, wie es beispielsweise in der WO 2008/ 028022 A2, auf dem der Oberbegriff des Anspruchs 1 basiert, und der US 2009/0143640 A1 beschrieben ist. Durch die vordefinierte Positionierung der Optik des Bildübertragungssystems innerhalb des expandierten Ballons werden dabei die für eine optimale Bildwiedergabe erforderlichen optischen Abstände zwischen aufzunehmendem Objekt und der Optik des Bildübertragungssystems erzielt. Durch Verschieben des Ballon-Laparoskops innerhalb des Bauchraums können aufeinander folgend unterschiedliche Bereiche sichtbar gemacht werden, ohne dass der gesamte Bauchraum mit Druckgas aufgepumpt zu werden braucht, wie dies bei der herkömmlichen diagnostischen Laparoskopie üblich ist.

Problematisch bei den eingangs genannten Ballon-Laparoskopen ist jedoch, dass bei der Verschiebung des Ballon-Laparoskops innerhalb des Bauchraums die Ballonwand oftmals an Organen oder Blutgefäßen zur Anlage kommt und bei einem weiteren Verschieben des Ballon-Laparoskops der Ballon an seiner Basis gegenüber dem rohrförmigen Körper abknicken kann. In diesem Fall sind die vordefinierten optischen Abstände zwischen Optik und aufzunehmendem Objekt nicht mehr gegeben, so dass die erforderliche Bildqualität nicht gewährleistet werden kann.

Da bei der Verwendung von Ballon-Laparoskopen keine Vollnarkose erforderlich ist, können diese in einfacher Weise für Nachkontrollen eingesetzt werden. Hierbei ist jedoch problematisch, dass die üblicherweise verwendeten Trokarhülsen nicht für eine längerfristige Verwendung ausgelegt sind und sich beispielsweise durch Bewegungen des Patienten lösen können. Auch sind grundsätzlich Schmerzen oder in seltenen Fällen innere Verletzungen bei entsprechend starken Bewegungen des Patienten durch die eingesetzten, in den Bauchraum ragenden Trokarhülsen nicht auszuschließen.

Es ist eine Aufgabe der vorliegenden Erfindung, ein Ballon-Laparoskop anzugeben, bei dem auch bei Bewegen des Laparoskops innerhalb der Bauchhöhle eine hohe Bildaufnahmequalität erzielbar ist. Weiterhin ist es eine Aufgabe der Erfindung, eine Trokarhülse anzugeben, die über einen längeren Zeitraum in die Bauchhöhle eingesetzt bleiben kann, ohne dass damit die beschriebenen Probleme verbunden sind.

Die das Ballon-Laparoskop betreffende Aufgabe wird ausgehend von einem Ballon-Laparoskop der eingangs genannten Art erfindungsgemäß dadurch gelöst, dass im Bereich der Befestigungsstelle zwischen dem Ballon und dem rohrförmigen Körper ein Stabilisierungselement für den Ballon vorgesehen ist, durch das ein Verschwenken des expandierten Ballons gegenüber dem rohrförmigen Körper verhindert oder zumindest erschwert wird.

Erfindungsgemäß wird somit dem beschriebenen Abknicken des Ballons an seiner proximal gelegenen Basis dadurch entgegengewirkt, dass in dem abknickgefährdeten Bereich ein Stabilisierungselement für den Ballon vorgesehen ist. Da der Ballon im übrigen Bereich aufgrund des Aufblasdrucks im expandierten Zustand eine relativ hohe Eigenstabilität besitzt, tritt das Abknicken üblicherweise ausschließlich im Übergangsbereich, d.h. an der Befestigungsstelle zwischen dem Ballon und dem rohrförmigen Körper auf. Daher wird durch die Anordnung eines Stabilisierungselements in diesem Bereich eine sehr hohe Stabilisierungswirkung erzielt.

Nach einer vorteilhaften Ausführungsform der Erfindung ist das Stabilisierungselement sowohl an dem rohrförmigen Körper als auch an dem Ballon befestigt. Durch die Befestigung an beiden Elementen, die bei dem beschriebenen Abknickvorgang eine Relativbewegung zueinander erfahren, kann eine sehr effektive Stabilisierung erreicht werden. Grundsätzlich ist es jedoch auch denkbar, dass das Stabilisierungselement nur an einem der beiden Teile, d.h. an dem rohrförmigen Körper oder an dem Ballon, insbesondere an dem rohrförmigen Körper, befestigt ist und sich das jeweils andere Teil bei einer Bewegung des Laparoskops an dem Stabilisierungselement abstützt, so dass ein Abknicken des Ballons verhindert wird.

Vorteilhaft ist das Stabilisierungselement auf der proximal gelegenen Seite des Ballons befestigt, da gerade an dieser Stelle die Abknicktendenz des Ballons am größten ist.

Weiter bevorzugt ist das Stabilisierungselement an der Außenseite des Ballons befestigt, insbesondere mit diesem verklebt oder verschweißt. Auf diese Weise wird durch das Stabilisierungselement die Dichtheit des Ballons nicht beeinträchtigt.

Nach einer weiteren vorteilhaften Ausführungsform der Erfindung umgreift das Stabilisierungselement einen proximalen Bereich des Ballons. Dadurch wird erreicht, dass das Stabilisierungselement gegen eine Knicktendenz in allen Richtungen gleichmäßig wirkt, da das Stabilisierungselement über den gesamten Umfang des Ballons in dessen proximalen Bereich eine Stabilisierung gewährleistet.

Bevorzugt besteht der Ballon aus einem flexiblen, dehnbaren Kunststoffmaterial, so dass er auf einen vordefinierten Arbeitsdurchmesser aufgeblasen werden kann. Das Stabilisierungselement besteht hingegen vorteilhaft aus einem flexiblen, im Wesentlichen nicht-dehnbaren Kunststoffmaterial. Durch die flexible Ausbildung kann das Stabilisierungselement im nicht expandierten Zustand des Ballons, ähnlich wie der Ballon selbst, zusammengefaltet werden, so dass er beim Einführen durch eine Trokarhülse kein Hindernis darstellt. Durch die nicht-dehnbare Ausbildung wird hingegen erreicht, dass im expandierten Zustand des Ballons das Stabilisierungselement seine Stabilisierungswirkung entfalten kann, da bei einer Krafteinwirkung auf den Ballon dieser durch die nicht-dehnbaren Stabilisierungselemente in seiner vordefinierten Position gehalten wird.

Nach einer weiteren vorteilhaften Ausführungsform der Erfindung wird das Stabilisierungselement beim Expandieren des Ballons vollständig gestreckt. Durch das vollständig gestreckte Stabilisierungselement wird erreicht, dass ein Abknicken des Ballons, das ein weiteres Strecken des Stabilisierungselements erforderlich machen würde, verhindert bzw. zumindest reduziert wird.

Vorteilhaft umfasst das Stabilisierungselement eine Wand aus Kunststofffolie. Insbesondere besitzt das Stabilisierungselement dabei bei expandiertem Ballon eine schalen- oder trichterförmige Form mit einer engen und einer gegenüberliegenden weiten Öffnung, wobei die enge Öffnung mit dem rohrförmigen Körper und die weite Öffnung mit dem Ballon verbunden sind. Durch diese schalen- oder trichterförmige Form ist eine optimale Anpassung des Stabilisierungselements sowohl an den expandierten Ballon mit relativ großem Umfang als auch an den rohrförmigen Körper mit kleinem Umfang möglich.

Nach einer weiteren vorteilhaften Ausführungsform der Erfindung sind in der Oberfläche des Stabilisierungselements eine oder mehrere Durchbrechungen ausgebildet. Dadurch ist eine Streckung des Stabilisierungselements in seine vollständig gestreckte Stellung problemlos möglich, da eine zwischen dem Ballon und dem Stabilisierungselement gebildete Kammer über die Durchbrechungen mit der Umgebung verbunden ist und somit bei Aufweiten der Kammer in dieser kein Unterdruck entsteht.

Nach einer weiteren bevorzugten Ausführungsform der Erfindung umfasst der rohrförmige Körper ein Außenrohr und ein Innenrohr, wobei sich das distale Ende des Innenrohrs über das distale Ende des Außenrohrs hinaus erstreckt. Bevorzugt ist dabei der Ballon an dem Innenrohr befestigt und vorteilhaft das proximale Ende des Stabilisierungselements an dem Außenrohr oder zwischen dem Außenrohr und dem Innenrohr befestigt.

Nach einer weiteren bevorzugten Ausführungsform der Erfindung ist das distale Ende des Außenrohrs trichterförmig aufgeweitet. Durch die trichterförmige Aufweitung kann eine Anpassung des Außenrohrs an das Stabilisierungselement erfolgen, wodurch die Stabilisierungswirkung weiter erhöht wird.

Bevorzugt erstreckt sich das Innenrohr durch das gesamte Außenrohr hindurch, so dass der rohrförmige Körper eine gleichmäßige Innenwand besitzt, durch die das Lumen des rohrförmigen Körpers in radialer Richtung begrenzt wird.

Nach einer weiteren bevorzugten Ausführungsform der Erfindung verläuft parallel zu dem rohrförmigen Körper ein zweites Rohr, das an dem rohrförmigen Körper befestigt und zum Einführen von minimal-invasiven Instrumenten ausgebildet ist. Über das zweite Rohr können somit beispielsweise Medikamente wie Anästhetika eingespritzt werden, wobei die Injektionsstelle gleichzeitig über das in dem rohrförmigen Körper angeordnete Laparoskop beobachtet werden kann. Anstelle von einer entsprechenden Injektionsnadel können auch sonstige minimal-invasive Instrumente, wie beispielsweise chirurgische Instrumente, insbesondere eine Biopsiezange oder ein Katheter zum Aspirieren und/ oder Saugen in die Bauchhöhle eingeführt werden. Durch das zweite Rohr ist nur ein einzelner Zugang zur Bauchhöhle erforderlich.

Bevorzugt ragt dabei das distale Ende des rohrförmigen Körpers über das distale Ende des zweiten Rohrs hinaus. Dadurch ist gewährleistet, dass das zweite Rohr die Expansion des Ballons nicht beeinträchtigt.

Vorteilhaft weist das distale Ende des zweiten Rohrs eine Austrittsöffnung auf, deren Öffnungsfläche schräg zur Längsachse des zweiten Rohrs verläuft. Insbesondere wenn die Austrittsöffnung von dem rohrförmigen Körper weg weist, ist gewährleistet, dass über das zweite Rohr eingeführte Instrumente an dem expandierten Ballon vorbei in die Bauchhöhle eingeführt werden können.

Der die Trokarhülse betreffende Teil der Aufgabe, der nicht Bestandteil der Erfindung ist, wird ausgehend von einer Trokarhülse der eingangs genannten Art dadurch gelöst, dass die Trokarhülse aus einem flexiblen Kunststoffmaterial besteht.

Durch die Ausbildung der Trokarhülse aus einem flexiblen Kunststoffmaterial wird erreicht, dass bei in der Bauchhöhle platzierter Trokarhülse Beschwerden des Patienten bei Bewegungen, insbesondere Schmerzen oder Verletzungen der inneren Organe, weitgehend ausgeschlossen werden können. Darüber hinaus wird durch die flexible Ausbildung erreicht, dass die im Inneren der Bauchhöhle auf die Trokarhülse wirkenden Kräfte bei Bewegung des Patienten von der flexiblen Trokarhülse aufgenommen werden können und somit eine Tendenz zum unerwünschten Herausschieben der eingesetzten Trokarhülse deutlich verringert wird.

Somit ist die Trokarhülse für eine längere Platzierung innerhalb der Bauchhöhle geeignet und kann daher für Nachkontrollen verwendet werden.

Nach einer Ausführungsform sind im Bereich des proximalen Endes der Trokarhülse ein oder mehrere Befestigungsmittel zum Befestigen der Trokarhülse an der Bauchdecke ausgebildet. Durch diese Befestigungsmittel wird eine sichere Fixierung der Trokarhülse erreicht, so dass auch bei stärkeren Bewegungen des Patienten ein unerwünschtes Herausschieben der Trokarhülse nicht möglich ist.

Vorteilhaft kann dazu im Bereich des proximalen Endes der Trokarhülse ein insbesondere umlaufender Flansch ausgebildet sein, an dem die Befestigungsmittel vorgesehen sind. Beispielsweise können die Befestigungsmittel als Ansätze ausgebildet sein, die mit der Bauchdecke verbindbar, insbesondere vemähbar sind. Vorteilhaft können die Ansätze dazu Löcher und/oder Vertiefungen zur Aufnahme von Fadenmaterial enthalten.

Nach einer weiteren Ausführungsform sind an der das Lumen der Trokarhülse begrenzenden Innenwand nach innen ragende längliche Gleitvorsprünge ausgebildet. Diese können beispielsweise in Längsrichtung der Trokarhülse oder auch spiralförmig verlaufen. Durch derartige Gleitvorsprünge wird die Reibung des durch die Trokarhülse in die Bauchhöhle einzuschiebenden Laparoskops oder sonstiger einzuführender Instrumente verringert.

Nach einer weiteren Ausführungsform ist an dem proximalen Ende der Trokarhülse ein Verbindungsmittel, insbesondere ein Gewinde ausgebildet. Über das Verbindungsmittel kann ein Verschlusseinsatz der Trokarhülse befestigt werden, so dass nach erfolgter Operation bzw. zwischen aufeinander folgenden Nachkontrollen die Trokarhülse jeweils sicher verschlossen werden kann.

Weiterhin wird ein nicht zur Erfindung gehörender Verschlusseinsatz für eine erfindungsgemäße Trokarhülse beschrieben, die einen flexiblen, lang gestreckten Mittelabschnitt aufweist, der sich vom proximalen bis zum distalen Ende der Trokarhülse erstreckt, wobei an dem distalen Ende des Mittelabschnitts ein Dichtungsabschnitt ausgebildet ist, dessen Außendurchmesser im Wesentlichen dem Innendurchmesser der Trokarhülse in deren distalem Endbereich entspricht.

Durch die Ausbildung des Verschlusseinsatzes bleibt die flexible Trokarhülse auch flexibel, wenn sie mit dem Verschlusseinsatz verschlossen ist. Somit bleibt der Vorteil der flexiblen Trokarhülse auch im verschlossenen Zustand erhalten. Bevorzug ist der Mittelabschnitt dabei elastisch ausgebildet.

Nach einer Ausführungsform ist im Bereich des proximalen Endes des Mittelabschnitts ein Verbindungsabschnitt zum lösbaren Verbinden des Verschlusselements mit der Trokarhülse ausgebildet. Insbesondere kann dabei der Verbindungsabschnitt ein Gewinde umfassen. Durch diese Ausbildung kann der Verschlusseinsatz an der Trokarhülse zuverlässig befestigt werden, so dass insbesondere die Dichtigkeit der Trokarhülse auch im verschlossenen Zustand gewahrt wird.

Bevorzugt umfasst der Mittelabschnitt einen Metalldraht, wobei der Metalldraht insbesondere mit einer Kunststoffschicht überzogen sein kann. Die Ausbildung mit einem Metalldraht gewährleistet zum einen die erforderliche Flexibilität und zum anderen eine stabile Ausbildung des Verschlusseinsatzes.

Der Dichtungsabschnitt und/oder der Verbindungsabschnitt können vorteilhaft aus Kunststoff bestehen. Dadurch ist eine kostengünstige Herstellung des Verschlusseinsatzes möglich.

Die Erfindung wird nachfolgend anhand von Ausführungsbeispielen unter Bezugnahme auf die Zeichnungen näher beschrieben; in diesen zeigen:
- Fig. 1: eine Seitenansicht eines erfindungsgemäß ausgebildeten Ballon-Laparoskops mit expandiertem Ballon,
- Fig. 2: eine Seitenansicht eines Mandrins,
- Fig. 3: eine Seitenansicht einer zum Aufblasen des Ballons verwendbaren Spritze,
- Fig. 4: einen Längsschnitt durch das Ballon-Laparoskop aus Fig. 1 mit zusammengefaltetem Ballon,
- Fig. 5: den Längsschnitt nach Fig. 4 mit expandiertem Ballon,
- Fig. 6: eine Detailansicht aus Fig. 5,
- Fig. 7: eine vergrößerte Ansicht eines Außenrohrs des Ballon-Laparoskops aus Fig. 1,
- Fig. 8: eine perspektivische Darstellung des erfindungsgemäßen ausgebildeten Ballon-Laparoskops aus Fig. 1,
- Fig. 9: eine teilweise geschnittene Seitenansicht einer zweiten Ausführungsform eines erfindungsgemäßen Ballon-Laparoskops,
- Fig. 10: eine teilweise geschnittene Darstellung des Mandrins aus Fig. 2,
- Fig. 11: eine Trokarhülse mit Trokar,
- Fig. 12: die Trokarhülse aus Fig. 11 in gebogenem Zustand,
- Fig. 13: eine perspektivische Darstellung der Trokarhülse aus Fig. 11,
- Fig. 14: einen Verschlusseinsatz für die Trokarhülse nach Fig. 11,
- Fig. 15: die Trokarhülse nach Fig. 11 mit eingesetztem Trokar,
- Fig. 16: einen Längsschnitt durch einen Teil der Trokarhülse aus Fig. 11 und
- Fig. 17: einen Querschnitt entlang der Ebene A-A aus Fig. 16.

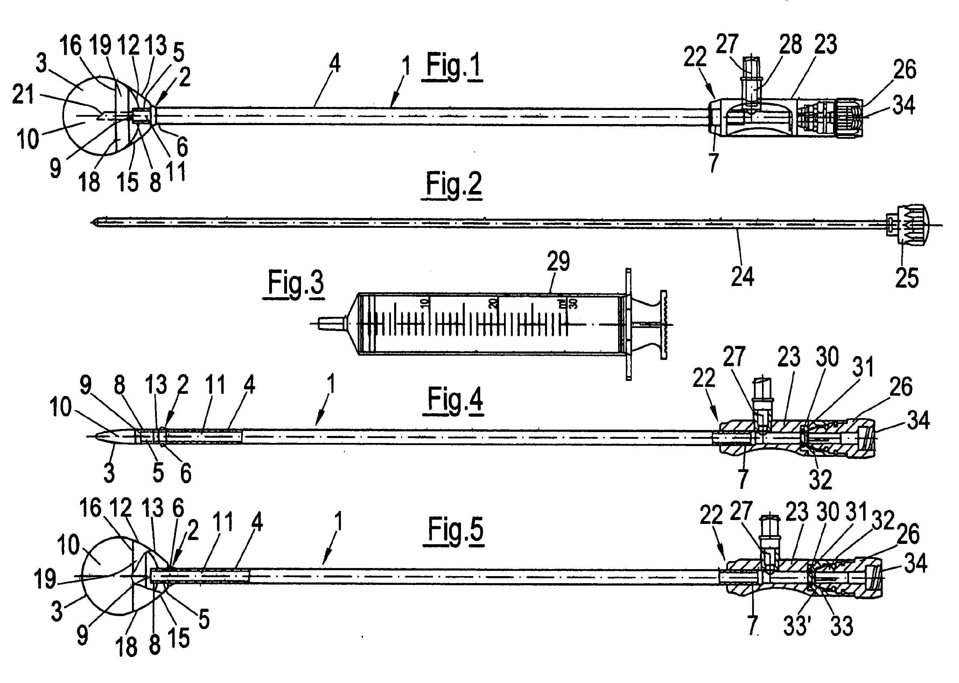

Das Ballon-Laparoskop gemäß Fig. 1 umfasst einen rohrförmigen Körper 1, an dessen distalen Endbereich 2 ein aufgeblasener Ballon 3 befestigt ist.

Der rohrförmige Körper 1 besteht aus einem Außenrohr 4 aus Metall sowie einem in das Außenrohr 4 eingesetzten Innenrohr 5, das aus Kunststoff besteht und sich über die gesamte Länge des Außenrohrs 4 erstreckt, wobei sich sowohl distal als auch proximal das Innenrohr 5 über das distale bzw. über das proximale Ende 6, 7 des Außenrohrs 4 hinaus erstreckt.

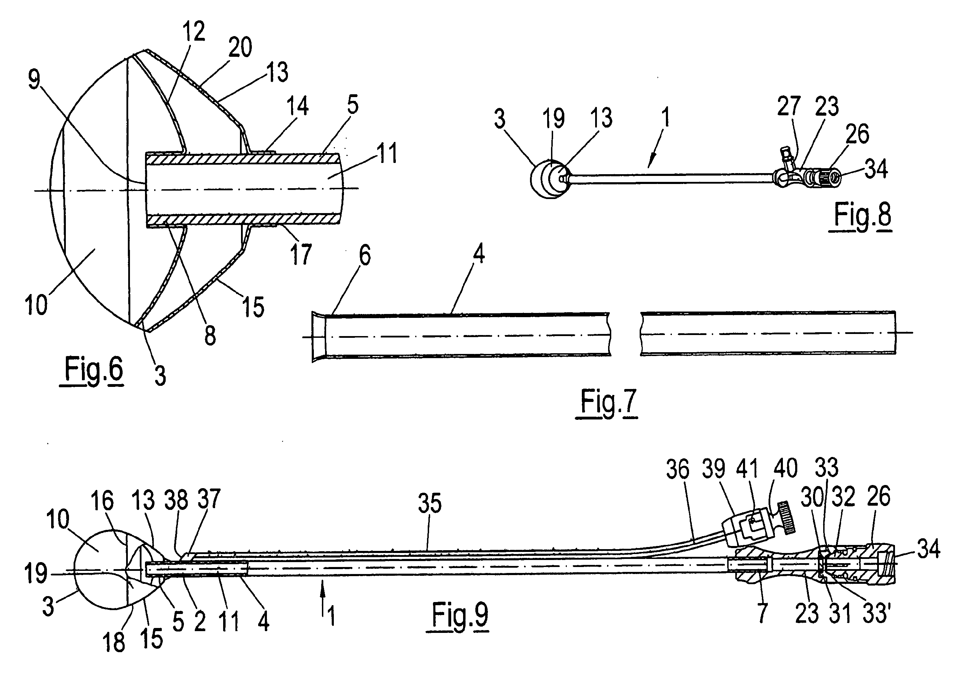

An dem überstehenden distalen Ende 8 des Innenrohrs 5 ist eine Öffnung 9 ausgebildet, die in dem Inneren 10 des Ballons 3 mündet und damit das Innere 10 des Ballons 3 mit dem Lumen 11 des rohrförmigen Körpers 1 verbindet. Dies ist aus der Schnittansicht nach Fig. 5 und insbesondere aus der Detailansicht nach Fig. 6 deutlicher zu erkennen.

Aus Fig. 6 ist ebenfalls gut zu erkennen, dass der proximale Bereich 12 des Ballons 3 an dem distalen Ende 8 des Innenrohrs 5 befestigt und insbesondere luftdicht mit diesem verklebt ist, so dass der Ballon 3 über das Lumen 11 des rohrförmigen Körpers 1 aufblasbar ist.

Weiterhin ist in den Fig. 1, 5 und 6 zu erkennen, dass am distalen Endbereich 2 des rohrförmigen Körpers 1 ein trichterförmiges Stabilisierungselement 13 vorgesehen ist, das über einen stutzenförmigen Ansatz 14 mit dem distalen Ende 6 des Innenrohrs 4 verbunden, insbesondere verklebt ist. Dabei liegt die Befestigungsstelle des stutzenförmigen Ansatzes 14 proximal zur Befestigungsstelle des Ballons 3, wie es aus Fig. 6 deutlich erkennbar ist.

An den stutzenförmigen Ansatz 14 schließt sich ein schalenförmiger Wandabschnitt 15 des Stabilisierungselements 13 an, der den proximalen Bereich 12 des Ballons 3 umgreift. Während der schalenförmige Wandabschnitt 15 in distaler Richtung in einer weiten Öffnung 16 des trichterförmigen Stabilisierungselements 13 endet, wird gegenüberliegend durch den stutzenförmigen Ansatz 14 eine enge Öffnung 17 des trichterförmigen Stabilisierungselements 13 gebildet.

Der schalenförmige Wandabschnitt 15 liegt mit seinem distalen Endbereich 18 an der Außenseite des Ballons 3 in dessen proximalen Bereich 12 an und ist mit dieser über eine ringförmige Kontaktfläche 19 befestigt, beispielsweise verschweißt oder verklebt. In dem schalenförmigen Wandabschnitt 15 sind mehrere Durchbrechungen 20 ausgebildet, von denen in Fig. 6 eines dargestellt ist.

Über das Lumen 11 des rohrförmigen Körpers 1 ist eine Bildaufnahmevorrichtung 21 so in das Innere 10 des Ballons 3 einführbar, dass sie mit ihrem distalen Ende in etwa im Mittelpunkt des Ballons 3 angeordnet ist, wie es gestrichelt in Fig. 1 angedeutet ist.

An dem proximalen Endbereich 22 des rohrförmigen Körpers 1 ist ein Anschlusselement 23 befestigt, über das sowohl die Bildaufnahmevorrichtung 21 als auch ein in Fig. 2 dargestellter Mandrin 24 in das Lumen 11 des rohrförmigen Körpers 1 eingeführt und befestigt werden kann. Zur Befestigung besitzt der Mandrin 24 an seinem proximalen Ende ein Befestigungselement 25, das mit einem entsprechenden Gegenelement 26, die insbesondere als Luerlock-Verbindung ausgebildet sein kann, verschraubbar ist.

Weiterhin ist das Lumen 11 des rohrförmigen Körpers 1 mit einem seitlichen Anschlusskanal 27 verbunden, in dem ein nicht näher dargestelltes Rückschlagventil 28 angeordnet ist.

An den Anschlusskanal 27 kann eine übliche Spritze 29 gemäß Fig. 3 angeschlossen werden, über die Fluid, beispielsweise in Form von Luft, über das Lumen 11 des rohrförmigen Körpers 1 in das Innere 10 des Ballons 3 eingebracht werden kann, wodurch dieser ausgeblasen wird.

Um den Ballon 3 aufblasen zu können, muss dabei das Lumen 11 des rohrförmigen Körpers 1 innerhalb des Anschlusselements 23 abgedichtet sein. Diese Abdichtung wird zum einen durch einen O-Ring 30 erreicht, der innerhalb eines Hohlraums 31 in dem Anschlusselement 23 angeordnet ist (siehe Fig. 4 und 5) und der bei eingeführter Bildaufnahmevorrichtung 21 an deren Außenfläche dichtend zur Anlage kommt.

Ist diese dichtende Anlage nicht ausreichend, so kann in einer nicht dargestellten Ausführungsform bei Einschrauben des als separates Teil ausgebildeten Gegenelements 26 in das Anschlusselement 23 der O-Ring 30 so zusammengequetscht werden, dass sowohl in radialer Richtung nach außen wie auch nach innen eine Querschnittsvergrößerung des O-Rings 30 erfolgt und dieser dadurch dichtend an der Außenfläche der Bildaufnahmevorrichtung 21 zur Anlage kommen.

Zusätzlich umfasst das Gegenelement 26 in seinem distalen Bereich mehrere in Umfangsrichtung verteilte Klemmabschnitte 32, die beim Einschrauben des Gegenelements 26 mit Anlaufschrägen 33 an entsprechende Anlaufschrägen 33' des Anschlusselements 23 auflaufen und dadurch radial nach innen gedrückt werden, bis sie ebenfalls zur Anlage an der Außenseite der Bildaufnahmevorrichtung 21 anliegen und dort die Abdichtung verstärken.

Aus Fig. 4 ist zu erkennen, dass der Ballon 3 im nicht expandierten Zustand so weit zusammengefaltet werden kann, dass sein Außendurchmesser im Wesentlichen dem Außendurchmesser des rohrförmigen Körpers 1 entspricht. Dazu ist die Wand des Ballons 3 aus einer elastischen, dünnen Kunststofffolie ausgebildet, die insbesondere im expandierten Zustand durchsichtig ausgebildet ist, um über die Bildaufnahmevorrichtung 21 eine Beobachtung der Umgebung zu ermöglichen.

Auch das Stabilisierungselement 13 ist aus einem dünnen folienartigen Kunststoffmaterial ausgebildet, das jedoch im Gegensatz zum Ballonmaterial nicht oder nur sehr wenig dehnbar ist. Durch diese Ausbildung kann auch das Stabilisierungselement 13 in dem in Fig. 4 dargestellten nicht expandierten Zustand des Ballons 3 so zusammengefaltet sein, dass der gesamte Außendurchmesser des Ballon-Laparoskops in seinem distalen Endbereich 2 im Wesentlichen durch den Außendurchmesser des rohrförmigen Körpers 1 bestimmt wird.

Aus Fig. 7 ist erkennbar, dass das distale Ende 6 des Außenrohrs 4 trichterförmig aufgeweitet ist, um sich an den schalenförmigen Wandabschnitt 15 des Stabilisierungselements 13 anzupassen und eine zusätzliche erhöhte Stabilisierung zu erreichen.

Das erfindungsgemäße Ballon-Laparoskop wird wie folgt verwendet:

Zum Einführen des Ballon-Laparoskops durch eine zuvor eingesetzte Trokarhülse wird zunächst der Mandrin 24 über eine proximale Öffnung 34 des Gegenelements 26 in das Lumen 11 des rohrförmigen Körpers vollständig eingeschoben und mit seinem Befestigungselement 25 mit dem Gegenelement 26 verschraubt. Der Ballon 3 befindet sich dabei in seinem in Fig. 4 dargestellten nicht expandierten Zustand und wird durch das distale Ende des eingeschobenen Mandrins 24 in die Länge gestreckt, um einen minimalen Durchmesser des Ballons 3 sowie des Stabilisierungselements 13 zu erreichen.

Nach Einführen des Ballon-Laparoskops wird der Mandrin 24 entnommen und anschließend die Bildaufnahmevorrichtung 21 über die proximale Öffnung 34 und das Lumen 11 des rohrförmigen Körpers 1 vollständig eingeschoben, bis das distalen Ende der Bildaufnahmevorrichtung 21 die in Fig. 1 gezeigte Position erreicht hat. In dieser Stellung wird die Bildaufnahmevorrichtung durch Verschrauben des Gegenelements 26 über die Klemmabschnitte 32 fixiert, wobei gleichzeitig über die Klemmabschnitte 32 und den O-Ring 30 die zuvor beschriebene Abdichtung im proximalen Bereich des Ballon-Laparoskops erfolgt.

Anschließend wird die Spitze 29 mit dem Anschlusskanal 27 verbunden und mittels der Spitze 29 Luft über den Anschlusskanal 27 in das Lumen 11 des rohrförmigen Körpers 1 eingebracht. Die Luft wird über einen zwischen der Außenseite der Bildaufnahmevorrichtung 21 und der Innenwand des Innenrohrs 5 gebildeten Ringkanal bis in das Innere 10 des Ballons 3 gedrückt, wodurch dieser in seinen expandierten Zustand gemäß Fig. 1 gebracht wird. Anschließend wird die Spitze 29 von dem Anschlusskanal 27 getrennt, wobei durch das Rückschlagventil 28 ein Ausströmen der Luft aus dem Anschlusskanal 27 verhindert wird und der Ballon 3 in seinem expandierten Zustand verbleibt.

In diesem Zustand kann das Ballon-Laparoskop innerhalb der Bauchhöhle nun bewegt und verschwenkt werden, um an die gewünschte Beobachtungsposition gebracht zu werden. Trifft bei einer solchen Bewegung die Außenwand des Ballons 3 auf ein Hindernis, so wird der Ballon 3 nicht oder nur unwesentlich gegenüber dem rohrförmigen Körper 1 abgeknickt, da beim Aufblasen des Ballons 3 der schalenförmige Wandabschnitt 15 des Stabilisierungselements 13 vollständig gestreckt wird und dadurch einer Abknickbewegung des Ballons 3 entgegenwirkt. Da das Stabilisierungselement 13 den Ballon 3 in seinem proximalen Bereich 12 vollständig umgreift, wird diese Stabilisierungswirkung für Abknickbewegungen des Ballons 3 in allen Richtungen erreicht. Grundsätzlich muss dabei das Stabilisierungselement 13 nicht einen vollumfänglichen Wandabschnitt 15 umfassen, sondern dieser kann aus mehreren, beispielsweise streifenförmigen Teilabschnitten bestehen.

Durch das Stabilisierungselement 13 wird somit erreicht, dass die Position des Ballons 3 gegenüber der eingesetzten Bildaufnahmevorrichtung 21 auch bei Bewegungen des Ballon-Laparoskops innerhalb der Bauchhöhle im Wesentlichen immer unverändert bleibt.

Das in Fig. 9 dargestellte abgewandelte Ausführungsbeispiel entspricht vollständig dem Ausführungsbeispiel gemäß den Fig. 1 bis 8, wobei zusätzlich an dem Außenrohr 4 ein zweites Rohr 35 befestigt ist, das im Wesentlichen parallel zu dem rohrförmigen Körper 1 verläuft und über das minimal-invasive Instrumente eingeführt werden können.

Wie in Fig. 9 gezeigt ist, kann zum Einführen des Ballon-Laparoskops zunächst ein Mandrin 36 in das zweite Rohr 35 eingeführt sein, um während des Einführens des Ballon-Laparoskops das zweite Rohr 35 zu verschließen.

Der Mandrin 36 besitzt dazu an seinem distalen Ende einen Dichtabschnitt 37, dessen Außendurchmesser im Wesentlichen gleich dem Innendurchmesser des zweiten Rohrs 35 ist. Weiterhin ist aus Fig. 9 zu erkennen, dass am distalen Ende des zweiten Rohrs eine Austrittsöffnung 38 ausgebildet ist, deren Öffnungsfläche schräg zur Längserstreckung des zweiten Rohrs 35 ausgerichtet ist. Dadurch wird erreicht, dass beim Einführen von minimal-invasiven Instrumenten diese an dem expandierten Ballon 3 vorbeigeführt werden und somit diesen nicht beschädigen. Dabei wird durch das Stabilisierungselement 13 auch bei einem Anliegen der über das zweite Rohr 35 eingeführten Instrumente an der Außenseite des Ballons 3 dieser in seiner korrekten Position gehalten, so dass der optische Abstand zwischen dem distalen Ende der Bildaufnahmevorrichtung 21 und der Wand des Ballons 3 eingehalten wird.

An dem proximalen Ende des zweiten Rohrs 35 ist ein Befestigungsabschnitt 39 ausgebildet, mit dem ein am Ende des Mandrins 36 befestigtes Verriegelungselement 40, beispielsweise über einen Bajonettverschluss 41 befestigt werden kann.

Anstelle von minimal-invasiven Instrumenten, wie beispielsweise Biopsiezangen, kann über das zweite Rohr 35 beispielsweise auch ein Katheter zum Aspirieren oder Saugen eingeführt werden. Es ist auch möglich, über einen entsprechenden flexiblen Katheter eine Injektionsnadel einzuführen, die beispielsweise am Ende eines weiteren flexiblen Schlauchs fest angebracht ist. Über diese Injektionsnadel kann z.B. Adrenalin zum Blutstillen eingespritzt werden. Beim Einführen ist es dabei vorteilhaft, wenn die Nadel in den flexiblen Schlauch zurückgezogen ist, um beim Austreten des distalen Endes des flexiblen Schlauchs aus der Öffnung 38 eine Verletzung des Ballons 3 zu vermeiden.

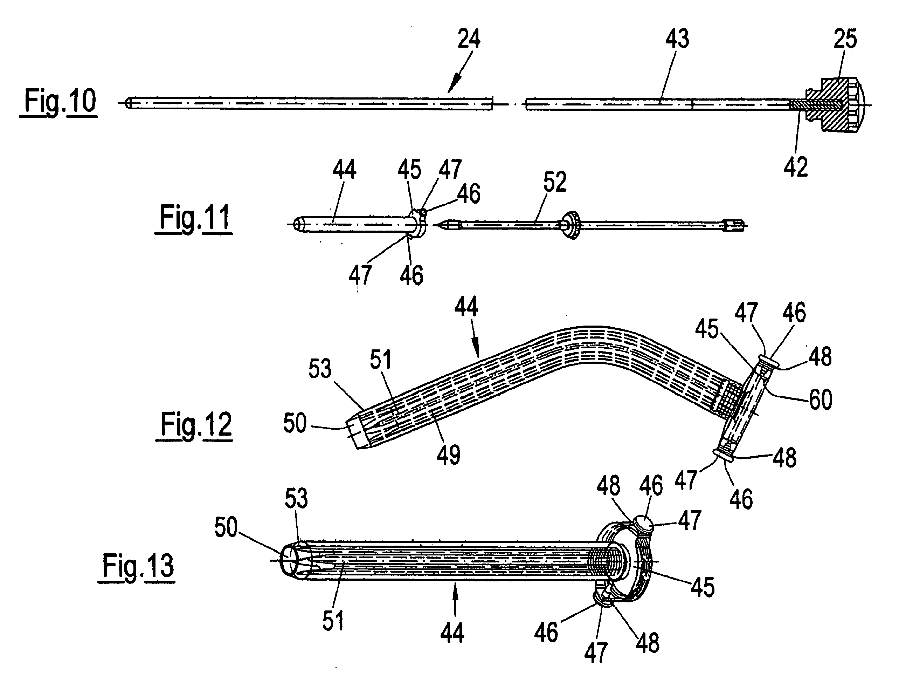

Aus der teilweise geschnittenen Ansicht des Mandrins 24 in Fig. 10 ist zu erkennen, dass dieser einen innen liegenden flexiblen Metalldraht 42 umfasst, der von einer Kunststoffhülle 43 ummantelt ist. Dadurch wird gleichzeitig eine stabile, flexible, bioverträgliche und an den Durchmesser des Lumens 11 angepasste Ausführungsform des Mandrins 24 geschaffen.

In Fig. 11 ist eine Trokarhülse 44 dargestellt, die aus flexiblem Kunststoffmaterial besteht. Wie insbesondere auch aus den Fig. 12, 13 und 15 erkennbar ist, ist an dem proximalen Ende der Trokarhülse 44 ein umlaufender Flansch 45 ausgebildet, an dem zwei radial gegenüberliegende, als Ansätze 46 ausgebildete Befestigungsmittel 47 ausgebildet sind. Der umlaufende Flansch 45 sowie die Ansätze 46 können dabei einstückig mit dem übrigen Teil der Trokarhülse 44 oder beispielsweise aus einem härteren Material als die Trokarhülse ausgebildet sein.

Im Bereich zwischen den Ansätzen 46 und dem Flansch 45 sind insbesondere umlaufende Ringnuten 48 ausgebildet, in die ein Faden zum Vernähen der Ansätze 46 mit der Bauchdecke eines Patienten eingelegt werden kann. Auf diese Weise ist es möglich, die Trokarhülse 44 über einen gewissen Zeitraum fest mit der Bauchdecke des Patienten zu verbinden,

Im Bereich zwischen den Ansätzen 46 und dem Flansch 45 sind insbesondere umlaufende Ringnuten 48 ausgebildet, in die ein Faden zum Vernähen der Ansätze 46 mit der Bauchdecke eines Patienten eingelegt werden kann. Auf diese Weise ist es möglich, die Trokarhülse 44 über einen gewissen Zeitraum fest mit der Bauchdecke des Patienten zu verbinden, um dadurch eine postoperative Untersuchung über mehrere Tage zu erleichtern.

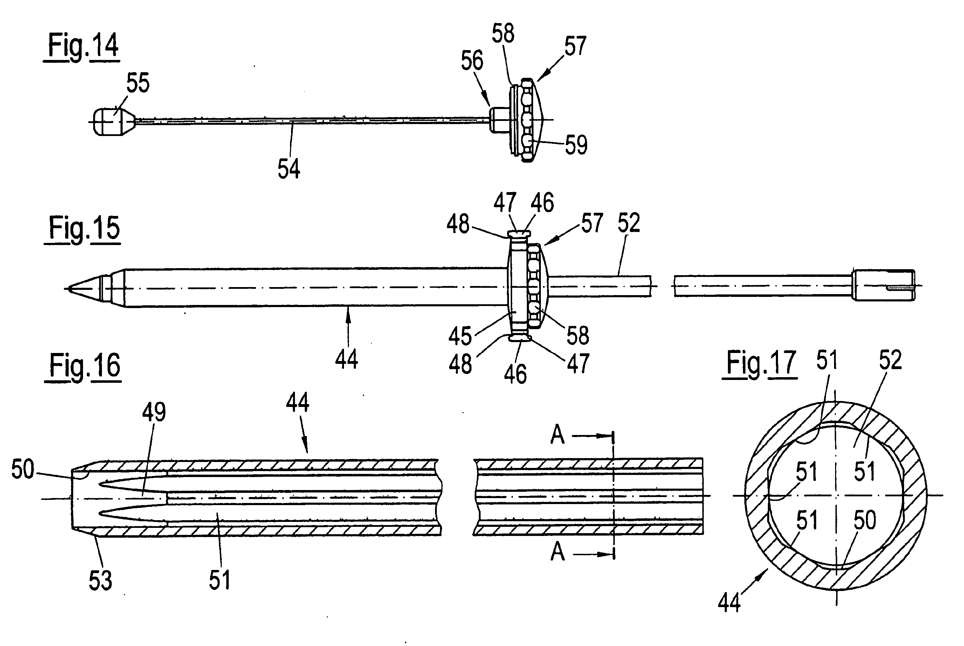

Die Trokarhülse 44 umfasst ein Lumen 49, das durch eine Innenwand 50 der Trokarhülse 44 begrenzt wird. An der Innenwand sind längliche Gleitvorsprünge 51 ausgebildet, so dass der Querschnitt des Lumens 49 nicht kreisförmig ausgebildet ist, sondern eine Form besitzt, die gewährleistet, dass ein einen kreisförmigen Querschnitt aufweisender, in die Trokarhülse 44 eingesetzter Trokar 52 nur über verringerte Kontaktflächen, insbesondere über im Wesentlichen linienförmige Kontaktstellen, mit seiner Außenseite an der Innenwand 50 der Trokarhülse 44 anliegt, wie es aus Fig. 17 zu erkennen ist. Dadurch wird die Reibung zwischen dem Trokar 52 und der Trokarhülse 44 beim Einführen des Trokars 52 verringert. In Fig. 17 sind die Gleitvorsprünge 51 durch ebene Wandabschnitte gebildet. Grundsätzlich können jedoch auch noch deutlicher ausgebildete Vorsprünge vorhanden sein.

Im distalen Endbereich 53 der Trokarhülse 44 sind keine Gleitvorsprünge 51 vorgesehen, damit eine Abdichtung zwischen der Innenwand 50 und dem eingesetzten Trokar 52 erreicht wird. Darüber hinaus ist die Trokarhülse 44 im distalen Endbereich 53 an ihrer Außenseite abgeschrägt ausgebildet, um das Einsetzen der Trokarhülse 44 zu erleichtern.

Um ein Verschließen der Trokarhülse 44 nach Entnahme des Trokars 52 zu ermöglichen, ist gemäß Fig. 14 ein Verschlusseinsatz vorgesehen, der einen flexiblen, lang gestreckten Mittelabschnitt 54 umfasst, der sich vom proximalen zum distalen Ende der Trokarhülse 44 erstreckt. Der Mittelabschnitt 54 kann dabei beispielsweise durch einen elastischen Metalldraht gebildet werden, der von einer Kunststoffumhüllung umgeben ist.

An dem distalen Ende des Mittelabschnitts 54 ist ein aus Kunststoff bestehender Dichtungsabschnitt 55 befestigt, dessen Außendurchmesser an den Innendurchmesser des distalen Endbereichs 53 der Trokarhülse 44 angepasst ist, so dass bei in die Trokarhülse 44 eingesetztem Verschlusseinsatz eine weitgehende Abdichtung der Trokarhülse 44 in ihrem distalen Endbereich 53 erfolgt.

An dem proximalen Ende 56 des Mittelabschnitts 54 ist ein Verbindungsabschnitt 57 befestigt, der ein Gewinde 58 sowie einen Betätigungsabschnitt 59 umfasst. Über das Gewinde 58 kann der Verschlusseinsatz mit einem entsprechenden Innengewinde 60, das an dem Flansch 45 der Trokarhülse 44 ausgebildet ist, verschraubt werden, so dass der in die Trokarhülse 44 eingesetzte Verschlusseinsatz sicher an der Trokarhülse 44 fixiert werden kann. Somit ist auch bei einem längeren Festlegen der Trokarhülse 44 an der Bauchdecke gewährleistet, dass zum einen eine sichere Abdichtung der Trokarhülse 44 erreicht wird und zum anderen in die Trokarhülse 44 kein Blut eindringen kann, das zu entsprechenden Verklumpungen innerhalb der Trokarhülse 44 führen könnte.

Durch die flexible Ausbildung der Trokarhülse 44 wird erreicht, dass auch bei einem länger dauernden Einsatz der Trokarhülse 44 keine Schmerzen oder Verletzungen innerhalb der Bauchhöhle des Patienten auftreten können. Die sichere Fixierung der Trokarhülse 44 wird dabei durch die beschriebenen Befestigungsmittel 47 erreicht.

### Bezugszeichenliste

- 1: rohrförmiger Körper
- 2: distaler Endbereich des rohrförmigen Körpers
- 3: Ballon
- 4: Außenrohr
- 5: Innenrohr
- 6: distales Ende des Außenrohrs
- 7: proximales Ende des Außenrohrs
- 8: distales Ende des Innenrohrs
- 9: distale Öffnung des Innenrohrs
- 10: Inneres des Ballons
- 11: Lumen des rohrförmigen Körpers
- 12: proximaler Bereich des Ballons
- 13: Stabilisierungselement
- 14: stutzenförmiger Ansatz
- 15: schalenförmiger Wandabschnitt
- 16: weite Öffnung
- 17: enge Öffnung
- 18: distaler Endbereich des schalenförmigen Wandabschnitts
- 19: ringförmige Kontaktfläche
- 20: Durchbrechungen
- 21: Bildaufnahmevorrichtung
- 22: proximaler Endbereich des rohrförmigen Körpers
- 23: Anschlusselement
- 24: Mandrin
- 25: Befestigungselement
- 26: Gegenelement
- 27: Anschlusskanal
- 28: Rückschlagventil
- 29: Spritze
- 30: O-Ring
- 31: Hohlraum
- 32: Klemmelemente
- 33, 33': Anlaufschrägen
- 34: proximale Öffnung des Gegenelements
- 35: zweites Rohr
- 36: Mandrin
- 37: Dichtabschnitt
- 38: Austrittsöffnung
- 39: Befestigungsabschnitt
- 40: Verriegelungselement
- 41: Bajonettverschluss
- 42: Metalldraht
- 43: Kunststoffhülse
- 44: Trokarhülse
- 45: Flansch
- 46: Ansätze
- 47: Befestigungsmittel
- 48: Ringnut
- 49: Lumen
- 50: Innenwand
- 51: Gleitvorsprünge
- 52: Trokar
- 53: distaler Endbereich
- 54: Mittelabschnitt
- 55: Dichtungsabschnitt
- 56: proximales Ende
- 57: Verbindungsabschnitt
- 58: Gewinde
- 59: Betätigungsabschnitt
- 60: Innengewinde

## Patentansprüche

1. Ballon-Laparoskop für diagnostische und/oder minimal-invasive Eingriffe mit einem rohrförmigen Körper (1), an dessen distalen Endbereich (2) ein Ballon (3) befestigt ist, wobei das Innere des Ballons (3) mit dem Lumen (11) des rohrförmigen Körpers (1) in Fluidverbindung steht, so dass der Ballon (3) durch Zufuhr von Fluid über das Lumen (11) des rohrförmigen Körpers (1) in einen expandierten Zustand gebracht werden kann,
**dadurch gekennzeichnet,**
**dass** im Bereich der Verbindungsstelle zwischen dem Ballon (3) und dem rohrförmigen Körper (1) ein aus einem flexiblen Kunststoffmaterial gebildetes Stabilisierungselement (13) für den Ballon (3) vorgesehen ist, das am Ballon (3) befestigt ist und beim Expandieren des Ballons (3) vollständig gestreckt wird und durch das ein Verschwenken des expandierten Ballons (3) gegenüber dem rohrförmigen Körper (1) verhindert oder zumindest erschwert wird.

2. Ballon-Laparoskop nach Anspruch 1,
**dadurch gekennzeichnet,**
**dass** das Stabilisierungselement (13) sowohl an dem rohrförmigen Körper (1) als auch an dem Ballon (3) befestigt ist.

3. Ballon-Laparoskop nach Anspruch 1 oder 2,
**dadurch gekennzeichnet,**
**dass** das Stabilisierungselement (13) auf der proximal gelegenen Seite des Ballons (3) befestigt ist.

4. Ballon-Laparoskop nach zumindest einem der vorhergehenden Ansprüche,
**dadurch gekennzeichnet,**
**dass** das Stabilisierungselement (13) an der Außenseite des Ballons (3) befestigt, insbesondere mit diesem verklebt oder verschweißt ist.

5. Ballon-Laparoskop nach zumindest einem der vorhergehenden Ansprüche,
**dadurch gekennzeichnet,**
**dass** das Stabilisierungselement (13) einen proximalen Bereich (12) des Ballons (3) umgreift.

6. Ballon-Laparoskop nach zumindest einem der vorhergehenden Ansprüche,
**dadurch gekennzeichnet,**
**dass** der Ballon (3) aus einem flexiblen, dehnbaren Kunststoffmaterial besteht.

7. Ballon-Laparoskop nach zumindest einem der vorhergehenden Ansprüche,
**dadurch gekennzeichnet,**
**dass** das Stabilisierungselement (13) aus einem im Wesentlichen nicht-dehnbaren Kunststoffmaterial besteht.

8. Ballon-Laparoskop nach zumindest einem der vorhergehenden Ansprüche,
**dadurch gekennzeichnet,**
**dass** das Stabilisierungselement (13) einen Wandabschnitt (15) aus KunststoffTolie umfasst.

9. Ballon-Laparoskop nach zumindest einem der vorhergehenden Ansprüche,
**dadurch gekennzeichnet,**
**dass** das Stabilisierungselement (13) bei expandiertem Ballon (3) eine schalen- oder trichterförmige Form mit einer engen und einer gegenüberliegenden weiten Öffnung (16, 17) besitzt, wobei die enge Öffnung (17) mit dem rohrförmigen Körper (1) und die weite Öffnung (16) mit dem Ballon (3) verbunden ist.

10. Ballon-Laparoskop nach zumindest einem der vorhergehenden Ansprüche,
dadurch **gekennzeichne**t,
dass in der Oberfläche des Stabilisierungselements (13) eine oder mehrere Durchbrechungen (20) ausgebildet sind.

11. Ballon-Laparoskop nach zumindest einem der vorhergehenden Ansprüche,
**dadurch gekennzeichnet,**
**dass** der rohrförmige Körper (1) ein Außenrohr (4) und ein Innenrohr (5) umfasst und dass sich das distale Ende (8) des Innenrohrs (5) über das distale Ende (6) des Außenrohrs (4) hinaus erstreckt, und insbesondere
**dass** der Ballon (3) an dem Innenrohr (5) befestigt ist.

12. Ballon-Laparoskop nach Anspruch 11,
**dadurch gekennzeichnet,**
**dass** das proximale Ende des Stabilisierungselements (13) an dem Außenrohr (4) oder zwischen dem Außenrohr (4) und dem Innenrohr (5) befestigt ist.

13. Ballon-Laparoskop nach Anspruch 11 oder 12,
**dadurch gekennzeichnet ,**
**dass** das distale Ende (6) des Außenrohrs (4) trichterförmig aufgeweitet ist.

14. Ballon-Laparoskop nach Anspruch 11, 12 oder 13,
**dadurch gekennzeichnet,**
**dass** sich das Innenrohr (5) durch das gesamte Außenrohr (4) hindurch erstreckt.

15. Ballon-Laparoskop nach zumindest einem der vorhergehenden Ansprüche,
**dadurch gekennzeichnet,**
**dass** parallel zu dem rohrförmigen Körper (1) ein zweites Rohr (35) verläuft, das an dem rohrförmigen Körper (1) befestigt und zum Einführen von minimal-invasiven Instrumente ausgebildet ist, und insbesondere
**dass** das distale Ende des rohrförmigen Körpers (1) über das distale Ende des zweiten Rohrs (35) hinausragt.

16. Ballon-Laparoskop nach zumindest einem der vorhergehenden Ansprüche,
**dadurch gekennzeichnet,**
**dass** das distale Ende des zweiten Rohrs (35) eine Austrittsöffnung (38) aufweist, deren Öffnungsfläche schräg zur Längsachse des zweiten Rohrs (35) verläuft, wobei bevorzugt die Austrittsöffnung (38) von dem rohrförmigen Körper (1) weg weist.

## Claims

1. A balloon laparoscope for diagnostic procedures and/or minimally invasive procedures having a tubular body (1) to whose distal end region (2) a balloon (3) is fastened, wherein the interior of the balloon (3) is in fluid communication with the lumen (11) of the tubular body (1) such that the balloon (3) can be brought into an expanded state by supplying fluid via the lumen (11) of the tubular body (1),
**characterised in that**
a stabilisation element (13) for the balloon (3) is provided in the region of the connection site between the balloon (3) and the tubular body (1), said stabilisation element being formed from a flexible plastic material, being fastened to the balloon (3), and being completely extended on the expanding of the balloon (3) and with a pivoting of the expanded balloon (3) with respect to the tubular body (1) being prevented or at least made more difficulty by said stabilisation element.

2. A balloon laparoscope in accordance with claim 1,
**characterised in that**
the stabilisation element (13) is fastened both to the tubular body (1) and to the balloon (3).

3. A balloon laparoscope in accordance with claim 1 or claim 2,
**characterised in that**
the stabilisation element (13) is fastened to the proximal disposed side of the balloon (3).

4. A balloon laparoscope in accordance with at least one of the preceding claims,
**characterised in that**
the stabilisation element (13) is fastened to the outside of the balloon (3), is in particular bonded or welded thereto.

5. A balloon laparoscope in accordance with at least one of the preceding claims,
**characterised in that**
the stabilisation element (13) engages around a proximal region (12) of the balloon (3).

6. A balloon laparoscope in accordance with at least one of the preceding claims,
**characterised in that**
the balloon (3) comprises a flexible, stretchable plastic material.

7. A balloon laparoscope in accordance with at least one of the preceding claims,
**characterised in that**
the stabilisation element (13) comprises a substantially non-stretchable plastic material.

8. A balloon laparoscope in accordance with at least one of the preceding claims,
**characterised in that**
the stabilisation element (13) includes a wall section (15) of plastic film.

9. A balloon laparoscope in accordance with at least one of the preceding claims,
**characterised in that**,
with an expanded balloon (3), the stabilisation element (13) has a shell-like or funnel-like shape having a narrow opening and a wide opening (16, 17) disposed opposite thereto wherein the narrow opening (17) is connected to the tubular body (1) and the wide opening (16) is connected to the balloon (3).

10. A balloon laparoscope in accordance with at least one of the preceding claims,
**characterised in that**
one or more apertures (20) are formed in the surface of the stabilisation element (13).

11. A balloon laparoscope in accordance with at least one of the preceding claims,
**characterised in that**
the tubular body (1) includes an outer tube (4) and an inner tube (5); and **in that** the distal end (8) of the inner tube (5) extends beyond the distal end (6) of the outer tube (4); and in particular in that the balloon (3) is fastened to the inner tube (5).

12. A balloon laparoscope in accordance with claim 11,
**characterised in that**
the proximal end of the stabilisation element (13) is fastened to the outer tube (4) or between the outer tube (4) and the inner tube (5).

13. A balloon laparoscope in accordance with claim 11 or claim 12,
**characterised in that**
the dismal end (6) of the outer tube (4) is widened in the manner of a funnel.

14. A balloon laparoscope in accordance with claim 11, claim 12 or claim 13,
**characterised in that**
the inner tube (5) extends through the total outer tube (4).

15. A balloon laparoscope in accordance with at least one of the preceding claims,
**characterised in that**
a second tube (35) which is fastened to the tubular body (1) and is configured for the introduction of minimally invasive instruments extends in parallel to the tubular body (1); and in particular in that the distal end of the tubular body (1) projects beyond the distal end of the second tube (35).

16. A balloon laparoscope in accordance with at least one of the preceding claims,
**characterised in that**
the distal end of the second tube (35) has an outlet opening (38) whose opening area extends obliquely to the longitudinal axis of the second tube (35), with the outlet opening (38) preferably facing away from the tubular body (1).

## Revendications

1. Laparoscope à ballon pour interventions de diagnostic et/ou interventions à invasion minimale comprenant un corps de forme tubulaire (1) à la zone terminale distale (2) duquel est fixé un ballon (3), de sorte que l'intérieur du ballon (3) est en communication fluidique avec le lumen (11) du corps de forme tubulaire (1), de telle manière que le ballon (3) peut être amené dans un état en expansion par admission de fluide via le lumen (11) du corps de forme tubulaire (1),
**caractérisé en ce que**, dans la zone de l'emplacement de liaison entre le ballon (3) et le corps de forme tubulaire (1), il est prévu pour le ballon (3) un élément de stabilisation (13), formé en une matière plastique flexible, qui est fixé sur le ballon (3) et qui est mis complètement en extension lors de l'expansion du ballon (3), et au moyen duquel un pivotement du ballon (3) en expansion par rapport au corps de forme tubulaire (1) est empêché ou au moins rendu difficile.

2. Laparoscope à ballon selon la revendication 1,
**caractérisé en ce que** l'élément de stabilisation (13) est fixé aussi bien sur le corps de forme tubulaire (1) que sur le ballon (3).

3. Laparoscope à ballon selon la revendication 1 ou 2,
**caractérisé en ce que** l'élément de stabilisation (13) est fixé sur le côté du ballon (3) disposé de manière proximale.

4. Laparoscope à ballon selon l'une au moins des revendications précédentes,
**caractérisé en ce que** l'élément de stabilisation (13) est fixé sur la face extérieure du ballon (3), en particulier collé ou soudé avec celui-ci.

5. Laparoscope à ballon selon l'une au moins des revendications précédentes,
**caractérisé en ce que** l'élément de stabilisation (13) entoure une zone proximale (12) du ballon (3).

6. Laparoscope à ballon selon l'une au moins des revendications précédentes,
**caractérisé en ce que** le ballon (3) est en une matière plastique flexible susceptible d'être allongée.

7. Laparoscope à ballon selon l'une au moins des revendications précédentes,
**caractérisé en ce que** l'élément de stabilisation (13) est en une matière plastique sensiblement non susceptible d'être allongée.

8. Laparoscope à ballon selon l'une au moins des revendications précédentes,
**caractérisé en ce que** l'élément de stabilisation (13) comprend un tronçon de paroi (15) en un film de matière plastique.

9. Laparoscope à ballon selon l'une au moins des revendications précédentes,
**caractérisé en ce que** l'élément de stabilisation (13) possède, lorsque le ballon (3) est en expansion, une forme en coque ou une forme en entonnoir avec une ouverture étroite et avec une ouverture large opposée (16, 17), dans lesquels l'ouverture étroite (16) est reliée au corps de forme tubulaire (1), et l'ouverture large (16) est reliée au ballon (3).

10. Laparoscope à ballon selon l'une au moins des revendications précédentes,
**caractérisé en ce qu'**une ou plusieurs traversées (20) sont réalisées dans la surface de l'élément de stabilisation (13).

11. Laparoscope à ballon selon l'une au moins des revendications précédentes,
**caractérisé en ce que** le corps de forme tubulaire (1) comprend un tube extérieur (4) et un tube intérieur (5), et **en ce que** l'extrémité distale (8) du tube intérieur (5) s'étend au-delà de l'extrémité distale (6) du tube extérieur (4), et en particulier
**en ce que** le ballon (3) est fixé au tube intérieur (5).

12. Laparoscope à ballon selon la revendication 11,
**caractérisé en ce que** l'extrémité proximale de l'élément de stabilisation (13) est fixée sur son tube extérieur (4), ou bien entre le tube extérieur (4) et le tube intérieur (5).

13. Laparoscope à ballon selon la revendication 11 août 12,
**caractérisé en ce que** l'extrémité distale (6) du tube extérieur (4) est élargie en forme d'entonnoir.

14. Laparoscope à ballon selon la revendication 11, 12 ou 13, **caractérisé en ce que** le tube intérieur (5) s'étend à travers la totalité du tube extérieur (4).

15. Laparoscope à ballon selon l'une au moins des revendications précédentes,
**caractérisé en ce qu'**un second tube (35) s'étend parallèlement au corps de forme tubulaire (1), qui est fixé au corps de forme tubulaire (1), et qui est réalisé pour l'introduction d'instruments à invasion minimale,
et en particulier **en ce que** l'extrémité distale du corps de forme tubulaire (1) dépasse au-delà de l'extrémité distale du second tube (35).

16. Laparoscope à ballon selon l'une au moins des revendications précédentes,
**caractérisé en ce que** l'extrémité distale du second tube (35) comporte une ouverture de sortie (38) dont la surface ouverte s'étend en oblique par rapport à l'axe longitudinal du second tube (35), et l'ouverture de sortie (38) est tournée de préférence en éloignement du corps de forme tubulaire (1).
